# NEUE EUROPÄISCHE PATENTSCHRIFT

(11) **EP 2 991 593 B2**
(45) Veröffentlichungstag und Bekanntmachung des Hinweises auf die Entscheidung über den Einspruch: **17.06.2020**
(45) Hinweis auf die Patenterteilung: 22.03.2017
(21) Anmeldenummer: 14721349.0
(22) Anmeldetag: 30.04.2014
(51) Int. Cl.: A61F 2/915

(54) **MEDIZINISCHE VORRICHTUNG ZUR EINFUHR IN EIN KÖRPERHOHLORGAN**
MEDICAL DEVICE FOR INSERTING INTO A HOLLOW ORGAN OF THE BODY
DISPOSITIF MÉDICAL DESTINÉ À ÊTRE INTRODUIT DANS UN ORGANE CREUX DU CORPS

(30) Priorität: 03.05.2013 DE 102013104550
(43) Veröffentlichungstag der Anmeldung: 09.03.2016
(73) Patentinhaber: Acandis GmbH & Co. KG, 76327 Pfinztal (DE)
(72) Erfinder: CATTANEO, Giorgio, 76199 Karlsruhe (DE); KLOPP, David, 75196 Remchingen (DE); NAGL, Frank, 76137 Karlsruhe (DE)
(74) Vertreter: Kilchert, Jochen
(86) Internationale Anmeldenummer: PCT/EP2014/058862
(87) Internationale Veröffentlichungsnummer: WO 2014/177634

(56) Entgegenhaltungen:
- EP-A2- 1 523 959
- WO-A1-2012/101163
- WO-A2-2005/053577
- US-A1- 2011 202 122

## Beschreibung

Die Erfindung betrifft eine medizinische Vorrichtung zur Einfuhr in ein Körperhohlorgan. Derartige Vorrichtungen sind beispielsweise als Stent oder Thrombektomiedevice bekannt.

Stents oder Thrombektomiedevices werden zur Behandlung verschiedenartiger Erkrankungen des Blutgefäßsystems eingesetzt und weisen oft eine expandierbare Gitterstruktur auf. Die Gitterstruktur wird über einen Katheter an den Behandlungsort geführt. Dazu nimmt die Gitterstruktur einen radial komprimierten Zustand ein, so dass sie innerhalb des Katheterkanals vorgeschoben werden kann.

Um eine gute Zuführbarkeit zu gewährleisten, ist es zweckmäßig, wenn die Gitterstruktur zumindest im Zuführzustand eine relativ hohe axiale Steifigkeit aufweist. Damit wird vermieden, dass sich die Gitterstruktur bei einer axialen Kraftbeaufschlagung radial weitet und so die Reibung an der Katheterinnenwand erhöht wird. Eine erhöhte axiale Steifigkeit führt bei bekannten Stents oder Thrombektomiedevices zu einer Reduktion der queraxialen Flexibilität, so dass die Platzierung der Gitterstruktur in kleinen, eng gewundenen Gefäßen, wie sie beispielsweise im Bereich des zerebralen Blutgefäßsystems vorkommen, erschwert ist.

Grundsätzlich ist bei der Gestaltung von medizinischen Vorrichtungen, die eine expandierbare Gitterstruktur aufweisen, wie es bei Stents und Thrombektomiedevices vorgesehen ist, erforderlich, einen Kompromiss zwischen einer hohen queraxialen Flexibilität und einer ausreichenden Radialkraft zu finden. Die Radialkraft dient dazu, die Gitterstruktur radial aufzuweiten und gegen eine Gefäßwand abzustützen. Durch Dimensionsänderungen, beispielsweise der Stegquerschnitte, kann zwar die queraxiale Flexibilität erhöht werden, so dass die Vorrichtung bzw. die Gitterstruktur gut durch stark gewundene, kleine Blutgefäße geführt werden kann. Gleichzeitig lässt damit jedoch die radiale Expansionsfähigkeit bzw. die Radialkraft nach. Es besteht die Gefahr, dass die Radialkraft nicht mehr ausreicht, um die Gitterstruktur sicher im Blutgefäß zu

Die Druckschrift D1 offenbart einen Stent mit einer Gitterstruktur und Stegen, welche Zellen begrenzen. Durch eine unterschiedlichs Flexibilität der Stege wird beim Übergang vom komprimierten Zustand zum expandierten Zustand der Gitterstruktur eine Torsion herbeigeführt.

Die Aufgabe der Erfindung besteht darin, eine medizinische Vorrichtung zur Einfuhr in ein Körperhohlorgan anzugeben, die eine ausreichende Radialkraft zur Verankerung im Körperhohlorgan, eine gute axiale Steifigkeit zur Zuführung durch einen Katheter und eine hohe queraxiale Flexibilität aufweist, so dass die Vorrichtung gut durch stark gewundene, kleine Körperhohlgefäße geführt werden kann.

Erfindungsgemäß wird diese Aufgabe durch den Gegenstand des Patentanspruchs 1 gelöst.

So beruht die Erfindung auf dem Gedanken, eine medizinische Vorrichtung zur Einfuhr in ein Körperhohlorgan mit einer komprimierbaren und expandierbaren Gitterstruktur aus Stegen anzugeben, die durch Stegverbinder einstückig miteinander verbunden sind und geschlossene Zellen der Gitterstruktur begrenzen. Die Stegverbinder weisen jeweils eine Verbinderachse auf, die sich zwischen zwei in Längsrichtung der Gitterstruktur benachbarten Zellen erstreckt. Die Stegverbinder rotieren beim Übergang der Gitterstruktur vom Herstellzustand in einen komprimierten Zustand, so dass sich ein Winkel zwischen der Verbinderachse und einer Längsachse der Gitterstruktur beim Übergang der Gitterstruktur von einem vollständig expandierten Herstellzustand in einen teilexpandierten Zwischenzustand ändert, insbesondere erhöht.

Grundsätzlich kann die Gitterstruktur der erfindungsgemäßen medizinischen Vorrichtung einstückig ausgebildet sein. Die Stege der Gitterstruktur können beispielsweise durch laserschneidende Bearbeitung eines röhrchenförmigen Rohlings freigeschnitten sein. Die freigeschnittenen Bereiche bilden die Zellen, die durch die Stege begrenzt sind. Bei der Erfindung handelt es sich vorzugsweise um eine Gitterstruktur mit einem Closed-Cell-Design. Die Zellen sind also vollständig von Stegen umschlossen. Erfindungsgemäß weisen die Zellen eine im Wesentlichen rautenartige Grundform auf. Mit anderen Worten sind die Zellen erfindungsgemäß durch jeweils vier Stege begrenzt.

Die Stegverbinder, die einstückig einen Teil der Gitterstruktur bilden, können folglich jeweils vier Stege miteinander koppeln. Die Stegverbinder bilden im Wesentlichen Kreuzungsstellen der Stege.

Bei der Komprimierung bzw. Expansion der Gitterstruktur ändern sich die Höhe und die Breite der einzelnen Zellen der Gitterstruktur. Durch die Rotation der Stegverbinder wird der Grad der Änderung von Höhe und Breite der Zelle beeinflusst. Insbesondere ergibt sich durch die Rotation der Stegverbinder ein unterschiedliches, insbesondere sich dynamisch änderndes, Verhältnis zwischen Zellenhöhe und Zellenbreite. Dies führt zu einer vergleichsweise hohen Flexibilität der Gitterstruktur, insbesondere in queraxialer Richtung. Insbesondere ermöglicht die Stegverbinderrotation, dass sich die Gitterstruktur beim Hindurchführen durch enge Körperhohlorgane ovalisieren kann. Die Gitterstruktur, die zumindest abschnittsweise einen kreiszylinderförmigen Querschnitt aufweisen kann, kann also bei dem Durchführen durch ein gekrümmtes Gefäß zumindest lokal eine ovale Querschnittsgeometrie einnehmen. So ist die medizinische Vorrichtung auch in sehr kleine Körperhohlorgane bzw. Blutgefäße einführbar, die starke Gefäßkrümmungen aufweisen.

Der Winkel zwischen der Verbinderachse und der Längsachse im Herstellzustand und/oder in einem komprimierten Zuführzustand der Gitterstruktur kann höchstens 5°, insbesondere höchstens 4°, insbesondere höchstens 3°, insbesondere höchstens 2°, insbesondere höchstens 1°, betragen. In besonderen Ausgestaltungen der Erfindung ist vorgesehen, dass die Verbinderachse im Herstellzustand und/oder in einem komprimierten Zuführzustand der Gitterstruktur parallel zur Längsachse der Gitterstruktur ausgerichtet ist. Insbesondere kann die Verbinderachse im Herstellzustand parallel und in einem teilexpandierten Zwischenzustand der Gitterstruktur in einem Winkel zur Längsachse der Gitterstruktur ausgerichtet sein. Durch die Ausrichtung der Verbinderachse im Herstellzustand parallel zur Längsachse der Gitterstruktur ist gewährleistet, dass die Gitterstruktur eine hohe axiale Stabilität aufweist, da sich die in Längsrichtung benachbart angeordneten Zellen und Stege auf diese Weise gegenseitig stützen.

Im Allgemeinen wird durch die Rotation der Stegverbinder die Flexibilität der Gitterstruktur erhöht, insbesondere ohne die Stabilität im expandierten Zustand bzw. im implantierten Zustand, zu beeinträchtigen. Die Flexibilität ist also erhöht, ohne die Radialkraft zu reduzieren.

Die Rotation des Stegverbinders erfolgt vorzugsweise in einer Wandungsebene der Gitterstruktur. Die Gitterstruktur kann im Allgemeinen zumindest abschnittsweise zylinderförmig ausgebildet sein, wobei die Zylindermantelfläche die Wandungsebene definiert. Mit anderen Worten kann der Stegverbinder um eine Rotationsachse rotieren, die senkrecht auf der Längsachse der Gitterstruktur steht.

Bei einer bevorzugten Ausführungsform der erfindungsgemäßen medizinischen Vorrichtung ist die Verbinderachse im komprimierten Zustand, insbesondere in einem Zuführzustand, parallel zur Längsachse der Gitterstruktur ausgerichtet. Insbesondere kann vorgesehen sein, dass die Verbinderachse sowohl im Herstellzustand, als auch im komprimierten Zustand, insbesondere im Zuführzustand, parallel zur Längsachse der Gitterstruktur ausgerichtet ist. Konkret kann der Stegverbinder beim Übergang der Gitterstruktur vom Herstellzustand in den komprimierten Zustand, insbesondere den Zuführzustand, die Rotationsrichtung wechseln.

Der Zuführzustand entspricht einem komprimierten Zustand, wobei die Gitterstruktur einen Expansionsgrad aufweist, der ausreichend klein ist, um die Gitterstruktur in einem Zuführsystem zu halten. Der Expansionsgrad kann bei 10% oder weniger liegen. Auf diese Weise ist die Gitterstruktur gut in kleine Zuführsysteme, beispielsweise Katheter, einführbar. Generell ist vorgesehen, dass in Zwischenzuständen der Gitterstruktur, insbesondere zwischen dem Zuführzustand und dem Herstellzustand, die Stegverbinder jeweils eine Verbinderachse aufweisen, die in einem Winkel zur Längsachse der Gitterstruktur ausgerichtet ist.

Im Allgemeinen wird darauf hingewiesen, dass bei einer Bezugnahme auf einen Winkel zwischen der Längsachse der Gitterstruktur und der Verbinderachse des Stegverbinders im Rahmen der vorliegenden Anmeldung tatsächlich auf eine Projektion der Längsachse der Gitterstruktur in der Wandungsebene, also der Ebene der Stegverbinder, abgestellt wird. Der Winkel zwischen der Verbinderachse und der Längsachse der Gitterstruktur zeigt sich also insbesondere in einer Seitenansicht der medizinischen Vorrichtung bzw. der Gitterstruktur.

Vorzugsweise entspricht die Verbinderachse dem kürzesten Abstand zwischen zwei Zellenspitzen von in Längsrichtung am Stegverbinder gegenüberliegenden Zellen. Generell umfasst die Gitterstruktur eine Vielzahl von Zellen, die sowohl in Längsrichtung, als auch in Umfangsrichtung der Gitterstruktur angeordnet sind. Die Zellen sind durch Stege und Stegverbinder voneinander getrennt. Insbesondere trennen die Stegverbinder Zellen in Längsrichtung der Gitterstruktur voneinander. Der kürzeste Abstand zwischen zwei Zellen, die Längsrichtung der Gitterstruktur unmittelbar benachbart zueinander angeordnet sind, bildet die Verbinderachse. Die Verbinderachse verläuft also entlang einer Linie durch den Stegverbinder, die von einer ersten Zelle zu einer zweiten, in Längsrichtung benachbart angeordneten Zelle, verläuft. Die kürzeste Verbindungslinie zwischen den beiden Zellen durch den Stegverbinder entspricht der Verbinderachse.

Bei einer weiteren bevorzugten Ausführungsform der Erfindung umfassen in Längsrichtung benachbarte Zellen der Gitterstruktur jeweils eine Zellenspitze mit einer Krümmung. Die Krümmung kann ein Maximum bzw. einen Scheitelpunkt, aufweisen, der einen Endpunkt der Verbinderachse bildet. Im Allgemeinen können Stege, die in Umfangsrichtung der Gitterstruktur benachbart zueinander angeordnet sind, an ihren Längsenden in einem Stegverbinder zusammengeführt sein. Dabei kann sich zwischen in Umfangsrichtung benachbarten Stegen eine Krümmung bilden, die gleichzeitig eine Begrenzung für die Zelle darstellt. Insbesondere begrenzt die Krümmung eine Zellenspitze, insbesondere die Zellenspitze einer geschlossenen Zelle, die vorzugsweise eine rautenförmige Grundform aufweist. Die Ecken der Raute bilden im Wesentlichen die Zellenspitzen, wobei diese nicht notwendigerweise spitz im Sinne einer scharfen Begrenzung sind, sondern eine Krümmung aufweisen können. Die Krümmung kann im Allgemeinen parabelförmig ausgebildet sein, so dass sich ein Maximum ergibt. Das Maximum bzw. der Scheitelpunkt der Krümmung bildet vorzugsweise den Endpunkt der Verbinderachse.

In einer weiteren bevorzugten Ausführungsform der erfindungsgemäßen Vorrichtung schneidet die Verbinderachse zumindest in einem teilexpandierten Zustand der Gitterstruktur der Längsachse der Gitterstruktur in einem Schnittpunkt, der einen Rotationspunkt bildet, um welchen der Stegverbinder rotiert. Gleichzeitig kann der Rotationspunkt einen Symmetriepunkt bilden. Insbesondere kann vorgesehen sein, dass der Stegverbinder bezogen auf den Rotationspunkt punktsymmetrisch ausgebildet ist.

Grundsätzlich können jeweils vier Stege an einem Stegverbinder angeordnet sein, wobei jeweils ein erster und dritter Steg und ein zweiter und vierter Steg diametral gegenüberliegend angeordnet sind. Konkret kann ein erster Steg diametral einem dritten Steg und ein zweiter Steg diametral einen vierten Steg gegenüberliegend angeordnet sein. Die Stege können jeweils eine neutrale Faser aufweisen. Die neutrale Faser entspricht einer Linie bzw. einer Zone des Steglängsschnitts, deren Länge sich bei einer Verformung des Stegs nicht ändert. In bevorzugten Ausführungsformen ist vorgesehen, dass jeweils eine neutrale Faser des zweiten und des dritten Stegs senkrecht auf den neutralen Fasern des ersten und des dritten Stegs steht. Im Allgemeinen kann die neutrale Faser des zweiten Stegs senkrecht zur neutralen Faser des ersten Stegs ausgerichtet sein. Die neutrale Faser des vierten Stegs kann senkrecht zur neutralen Faser des dritten Stegs ausgerichtet sein. Grundsätzlich sind andere Winkel möglich. Beispielsweise können die neutralen Fasern einen Winkel zwischen zwei im Stegverbinder verbundenen Stegen einschließen, der höchstens 90°, insbesondere höchstens 75°, insbesondere höchstens 55°, insbesondere höchstens 50°, insbesondere höchstens 45°, beträgt.

Die neutralen Fasern des zweiten und des vierten Stegs können fluchtend zueinander angeordnet sein. Mit anderen Worten können der erste und der dritte Steg eine gemeinsame, durchgehende neutrale Faser aufweisen. Demgegenüber ist bevorzugt vorgesehen, dass die neutralen Fasern des zweiten und des vierten Stegs versetzt zueinander angeordnet sind. Mit anderen Worten können die neutralen Fasern des zweiten und des vierten Stegs in einem Abstand zueinander auf die neutrale Faser des ersten und des dritten Stegs treffen. Dies begünstigt die Rotation des Stegverbinders und somit die Flexibilität der Gitterstruktur.

Der zweite und der vierte Steg können jeweils eine verbreiterte Wurzel aufweisen. Die Stege weisen üblicherweise eine Breite auf, die über den Großteil der Steglänge konstant ist. Im Bereich des Stegverbinders, insbesondere beim Übergang zum Stegverbinder, können zumindest diametral gegenüberliegende Stege, nämlich der zweite und der vierte Steg, eine verbreiterte Wurzel aufweisen. Dies erhöht die Stabilität des Stegverbinders und insbesondere der gesamten Gitterstruktur.

Der zweite und vierte Steg können im Bereich des Stegverbinders einen S-förmigen Verlauf aufweisen. Der erste und dritte Steg, insbesondere deren neutrale Faser, kann im Wesentlichen einen geradlinigen Verlauf aufweisen. Der zweite und vierte Steg können insbesondere eine Biegung aufweisen, der in einen Endabschnitt, insbesondere einem abgewinkelten Endabschnitt, des Stegs übergeht und den Übergang in den Stegverbinder ermöglicht. Die Biegung bzw. der Knick bildet eine Verformungszone, die zur Komprimierung bzw. Expansion der Gitterstruktur beiträgt.

Insgesamt ergibt sich eine Expansion oder Komprimierung, d.h. allgemein eine radiale Querschnittsgrößenänderung, der Gitterstruktur durch eine Verformung der Stege der Gitterstruktur. Durch die Biegung bzw. den Knick an den Stegenden beim Übergang in den Stegverbinder werden definierte Stellen für die Verformung der Stege geschaffen, so dass eine Verformung der Stege gut vorhersehbar ist. Durch den ersten und dritten Steg, die im Wesentlichen miteinander fluchten, so dass sich ein im Wesentlichen gerader Stegverlauf ergibt, wird die Stabilität der Gitterstruktur verbessert.

In einer weiteren bevorzugten Ausführungsform kann der Stegverbinder eine taillierte Form aufweisen. Die taillierte Form zeigt sich insbesondere in einer Verjüngung des Querschnitts des Stegverbinders, wobei der Querschnitt vorzugsweise in Umfangsrichtung der Gitterstruktur gebildet wird. Durch die taillierte Ausführung des Stegverbinders wird die Flexibilität der Gitterstruktur weiter erhöht, insbesondere ohne negative Beeinflussung der Radialkraft.

Für eine hohe Biegeflexibilität und längsaxiale Stabilität der Gitterstruktur in einem Zuführzustand ist es bevorzugt, wenn die Verbinderachse bei einem Expansionsgrad der Gitterstruktur von 5% bis 15%, insbesondere von 8% bis 12%, insbesondere 10% oder 9.9%, unter einem Winkel von höchstens 5°, insbesondere höchstens 4°, insbesondere höchstens 3°, insbesondere höchstens 2°, insbesondere höchstens 1°, zur Längsachse der Gitterstruktur ausgerichtet ist. Die Verbinderachse kann bei einem Expansionsgrad der Gitterstruktur von 5% bis 15%, insbesondere von 8% bis 12%, insbesondere 10% oder 9.9%, auch parallel zur Längsachse der Gitterstruktur ausgerichtet sein.

Alternativ oder zusätzlich kann die Verbinderachse bei einem Expansionsgrad von mindestens 90%, insbesondere mindestens 95%, insbesondere mindestens 98%, insbesondere 100%, unter einem Winkel von höchstens 5°, insbesondere höchstens 4°, insbesondere höchstens 3°, insbesondere höchstens 2°, insbesondere höchstens 1°, zur Längsachse der Gitterstruktur ausgerichtet sein. Die Verbinderachse kann bei einem Expansionsgrad der Gitterstruktur von mindestens 90%, insbesondere mindestens 95%, insbesondere mindestens 98%, insbesondere 100%, auch parallel zur Längsachse der Gitterstruktur ausgerichtet sein. Der Expansionsgrad von 100% entspricht dem vollständig expandierten Herstellzustand der Gitterstruktur.

Hinsichtlich der Zwischenzustände der Gitterstruktur ist Folgendes vorgesehen:
Bei einem Expansionsgrad von 40% bis 50%, insbesondere von 42% bis 48%, insbesondere 44,4%, kann die Verbinderachse mit der Längsachse der Gitterstruktur einen Winkel von 15° bis 20°, insbesondere von 16° bis 19°, insbesondere 18°, einschließen.

Ferner kann die Verbinderachse bei einem Expansionsgrad von 60% bis 70%, insbesondere von 62% bis 68%, insbesondere 66,7%, mit der Längsachse der Gitterstruktur einen Winkel von 12° bis 18°, insbesondere von 13° bis 17,5°, insbesondere von 14° bis 17°, insbesondere 16°, einschließen.

Überdies ist es denkbar, wenn die Verbinderachse bei einem Expansionsgrad von 80% bis 90%, insbesondere von 82% bis 90%, insbesondere von 84% bis 89,5%, insbesondere von 86% bis 89%, insbesondere 88,9%, mit der Längsachse der Gitterstruktur einen Winkel von 5° bis 12°, insbesondere von 6° bis 11°, insbesondere von 7° bis 10°, insbesondere 8°, einschließt.

Die zuvor genannten Expansionsgrade und Expansiongradbereiche können mit den jeweils dzu korrelierenden Winkeln und Winkelbereichen beliebig kombiniert werden. So ist beispielsweise in besonders bevorzugten Ausführungsformen vorgesehen, dass die Verbinderachse bei einem Expansionsgrad der Gitterstruktur von 9,9% und/oder von 100% jeweils parallel zur Längsachse der Gitterstruktur ausgerichtet ist. Bei einem Expansionsgrad von 44,4% kann die Verbinderachse mit der Längsachse der Gitterstruktur einen Winkel von 18°, bei einem Expansionsgrad von 66,7% einen Winkel von 16° und bei einem Expansionsgrad von 88,9% einen Winkel von 8° einschließen.

Hinsichtlich der Gitterstruktur ist erfindungsgemäß vorgesehen, dass diese selbstexpandierbar ist. Konkret kann die Gitterstruktur ein superelastisches Material, insbesondere eine Nickel-Titan-ILegierung, vorzugsweise Nitinol, aufweisen. Die Gitterstruktur kann auch einstückig ausgebildet sein. Insbesondere kann die Gitterstruktur aus einem superelastischen Material, insbesondere einer Nickel-Titan-Legierung, beispielsweise Nitinol, bestehen. Superelastische Materialien der vorgenannten Art weisen eine hohe Elastizität auf, die die Rotation der Stegverbinder ermöglicht, insbesondere ohne eine plastische Verformung. Ferner stellen derartige superelastische Materialien eine ausreichende Radialkraft bereit, um die Gitterstruktur, auch gegen Widerstand einer Gefäßwand, radial aufzudehnen. Nickel-Titan-Legierungen haben außerdem Formgedächtniseigenschaften, die vorteilhaft zur Selbstexpandierbarkeit beitragen.

Hinsichtlich der Dimensionen der Gitterstruktur ist es vorteilhaft, wenn die Gitterstruktur im Herstellzustand einen Querschnittsdurchmesser aufweist, der zwischen 3,5 mm und 6 mm, insbesondere 3,5 mm oder 4,5 mm oder 6 mm, beträgt. Die vorgenannten Werte gelten für den Herstellzustand, in welchem die Gitterstruktur vollständig expandiert ist. Im implantierten Zustand weist die Gitterstruktur vorzugsweise einen Querschnittsdurchmesser auf, der um 0,5 mm bis 1,5 mm kleiner als im Herstellzustand ist. So ist gewährleistet, dass die Gitterstruktur eine ausreichende Radialkraft aufbringt, um sich in einem Körpergefäß zu verankern.

Ferner kann die Gitterstruktur in Umfangsrichtung jeweils zwischen drei und sechs, insbesondere zwischen drei und neun, vorzugsweise sechs, unmittelbar benachbarte Zellen aufweisen, die einen Zellenring bilden. Mit anderen Worten weist die Gitterstruktur in bevorzugten Ausführungsformen einen Zellenring von Zellen auf, wobei die Anzahl der Zellen auf die vorgenannten Werte bzw. Wertebereiche beschränkt ist. In weiteren Ausführungsformen kann vorgesehen sein, dass die Gitterstruktur in Umfangsrichtung jeweils mehr als zwölf, insbesondere zwischen zwölf und 48, unmittelbar benachbarte Zellen aufweist, die einen Zellenring bilden.

Generell weist die Gitterstruktur Zellen auf, die jeweils durch vier Stege begrenzt sind. Dabei können ein erster und dritter Steg jeweils eine erste Stegbreite und ein zweiter und vierter Steg jeweils eine zweite Stegbreite aufweisen. Das Verhältnis zwischen der ersten Stegbreite und der zweiten Stegbreite beträgt vorzugsweise zwischen 1:1,1 und 1:3, insbesondere zwischen 1:1,1 und 1:1,5, insbesondere zwischen 1:1,25 und 1:1,5, insbesondere zwischen 1:1,1 und 1:1,3, vorzugsweise 1:1,3, 1:1,25 oder 1:1,2. Das Stegbreitenverhältnis beeinflusst die Rotationsfähigkeit des Stegverbinders und gewährleistet ein ausreichendes radiales Expansionsvermögen der Gitterstruktur in stark gewundenen Gefäßanatomien.

Für ein optimales Stegbreitenverhältnis ist zudem die Anzahl der Zellen eines Zellenrings der Gitterstruktur zu berücksichtigen. So ist es bevorzugt, dass bei einer Gitterstruktur, die einen Zellenring mit drei bis neun Zellen, insbesondere sechs Zellen, aufweist, das Verhältnis zwischen der ersten Stegbreite und der zweiten Stegbreite vorzugsweise zwischen 1:1,1 und 1:1,5, insbesondere zwischen 1:1,1 und 1:1,3, vorzugsweise 1:1,3 beträgt. Bei einer Gitterstruktur, die einen Zellenring mit mehr als zwölf, insbesondere zwischen zwölf und 48, Zellen aufweist, beträgt das Verhältnis zwischen der ersten Stegbreite und der zweiten Stegbreite vorzugsweise zwischen 1:1,1 und 1:3, insbesondere 1:1,25 oder 1:1,2.

Die Erfindung wird im Folgenden anhand eines Ausführungsbeispiels unter Bezugnahme auf die beigefügten, schematischen Zeichnungen näher erläutert. Darin zeigen:
- Fig. 1:: eine Seitenansicht einer erfindungsgemäßen medizinischen Vorrichtung in einem Zuführzustand, insbesondere bei einem Expansionsgrad von 9,9%;
- Fig. 2:: eine Detailansicht eines Stegverbinders der medizinischen Vorrichtung gemäß Figur 1;
- Fig. 3:: eine Seitenansicht der medizinischen Vorrichtung gemäß Fig. 1 in einem teilexpandierten Zustand, insbesondere bei einem Expansionsgrad von 44,4%;
- Fig. 4:: eine Detailansicht eines Stegverbinders der medizinischen Vorrichtung gemäß Fig. 3;
- Fig. 5:: eine Seitenansicht der medizinischen Vorrichtung gemäß Fig. 1 in einem teilexpandierten Zustand, insbesondere bei einem Expansionsgrad von 66,7%;
- Fig. 6:: eine Detailansicht eines Stegverbinders der medizinischen Vorrichtung gemäß Fig. 5;
- Fig. 7:: eine Seitenansicht der medizinischen Vorrichtung gemäß Fig. 1 in einem teilexpandierten Zustand, insbesondere bei einem Expansionsgrad von 88,9%;
- Fig. 8:: eine Detailansicht eines Stegverbinders der medizinischen Vorrichtung gemäß Fig. 7;
- Fig. 9:: eine Seitenansicht der medizinischen Vorrichtung gemäß Fig. 1 im Herstellzustand, insbesondere bei einem Expansionsgrad von 100%;
- Fig. 10:: eine Detailansicht eines Stegverbinders der medizinischen Vorrichtung gemäß Fig. 9;
- Fig. 11:: ein Diagramm zur Darstellung der Winkel zwischen Stegverbinder und Längsachse der Gitterstruktur im Verhältnis zum Expansionsgrad bei der medizinischen Vorrichtung gemäß Fig. 1;
- Fig. 12:: eine Detailansicht eines Stegverbinders der medizinischen Vorrichtung gemäß Fig. 1 bei einem Expansionsgrad von 44,4%, wobei die neutralen Fasern der Stege dargestellt sind;
- Fig. 13:: die Detailansicht des Stegverbinders gemäß Fig. 12, wobei ein Versatz der Zellenspitzen in Längsrichtung benachbarter Zellen dargestellt ist; und
- Fig. 14:: die Detailansicht des Stegverbinders gemäß Fig. 12, wobei ein Versatz der neutralen Fasern diametral gegenüberliegender Stege dargestellt ist;

Die beigefügten Figuren zeigen eine medizinische Vorrichtung, die zur Einfuhr in ein Körperhohlorgan geeignet ist. Die medizinische Vorrichtung weist dazu insbesondere eine Gitterstruktur 10 auf, die komprimierbar und expandierbar ist. Mit anderen Worten kann die Gitterstruktur 10 einen Zuführzustand einnehmen, in dem die Gitterstruktur 10 einen relativ kleinen Querschnittsdurchmesser aufweist. Die Gitterstruktur 10 ist erfindungsgemäß selbstexpandierbar, so dass die Gitterstruktur 10 sich ohne Einfluss äußerer Kräfte selbsttätig auf einen maximalen Querschnittsdurchmesser aufweitet. Der Zustand, in dem die Gitterstruktur 10 den maximalen Querschnittsdurchmesser aufweist, entspricht dem Herstellzustand. Im Herstellzustand weist die Gitterstruktur 10 einen Expansionsgrad von 100% auf. Ein Expansionsgrad von 100% entspricht also dem maximalen Querschnittsdurchmesser, den die Gitterstruktur 10 selbsttätig einnehmen kann. In diesem Zustand übt die Gitterstruktur 10 keinerlei Radialkräfte aus.

Vorzugsweise ist die Gitterstruktur 10 einstückig ausgebildet. Insbesondere kann die Gitterstruktur 10 zumindest abschnittsweise zylinderförmig ausgebildet sein. Die Gitterstruktur 10 ist vorzugsweise aus einem rohrförmigen Rohling durch Laserschneiden hergestellt. Dabei werden einzelne Stege 11, 12, 13, 14 der Gitterstruktur durch die laserschneidende Bearbeitung freigelegt. Die aus dem Rohling entfernten Bereiche bilden Zellen 30 der Gitterstruktur 10.

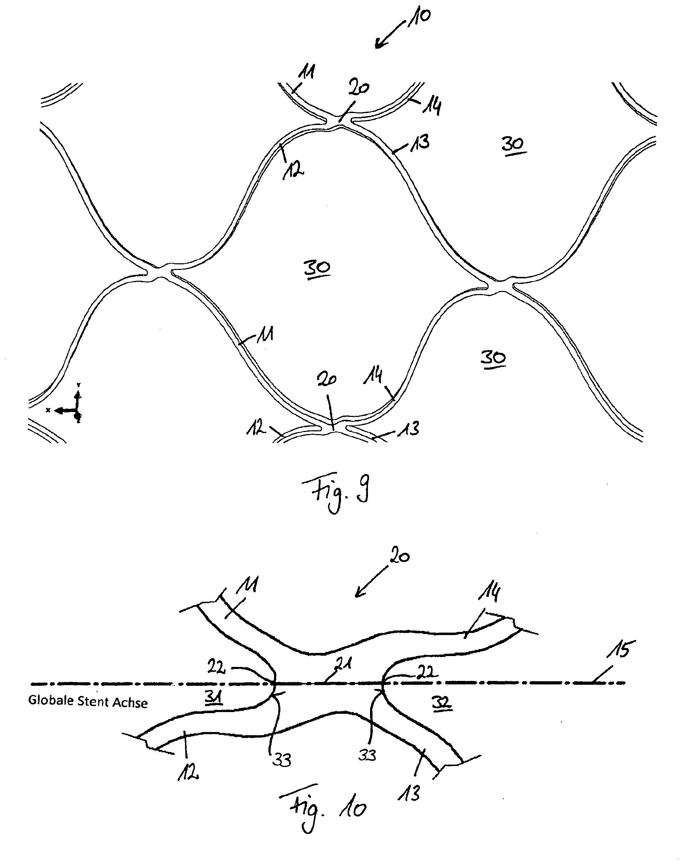

Die Zellen 30 weisen im Wesentlichen eine rautenförmige Grundform auf. Dies ist in Fig. 9 gut erkennbar. Insbesondere sind die Zellen 30 durch jeweils vier Stege 11, 12, 13, 14 begrenzt, wobei die Stege 11, 12, 13, 14 zumindest teilweise einen gekrümmten, insbesondere S-förmigen Verlauf aufweisen können. Die Zellen 30 weisen jeweils Zellenspitzen 31, 32 auf, die die Eckpunkte der rautenförmigen Grundform festlegen. Die Zellenspitzen 31, 32 sind jeweils an Stegverbindern 20 angeordnet, die jeweils vier Stege 11, 12, 13, 14 einstückig miteinander verbinden. Von jedem Stegverbinder 20 gehen jeweils vier Stege 11, 12, 13, 14 aus, wobei jeder Steg 11, 12, 13, 14 jeweils zwei Zellen 30 zugeordnet ist. Die Stege 11, 12, 13, 14 begrenzen jeweils die Zellen 30.

Die Gitterstruktur 10 kann grundsätzlich mehrere, unterschiedliche Zustände einnehmen, die sich durch den Expansionsgrad der Gitterstruktur 10 unterscheiden. Ein Expansionsgrad von 0% entspricht dabei einem theoretischen Querschnittsdurchmesser der Gitterstruktur 10 von 0 mm. In der Praxis ist ein derartiger Expansionsgrad nicht erreichbar. Der kleinstmögliche Expansionsgrad liegt in der Praxis vorzugsweise bei maximal 10%. In diesem maximal komprimierten Zustand wird die Gitterstruktur 10 durch einen Katheter an den Behandlungsort geführt. Im Rahmen der Anmeldung wird dieser Zustand als Zuführzustand bezeichnet. Bei den in den Figuren gezeigten Ausführungsbeispielen weist die Gitterstruktur 10 im Zuführzustand einen Expansionsgrad von 9,9% auf.

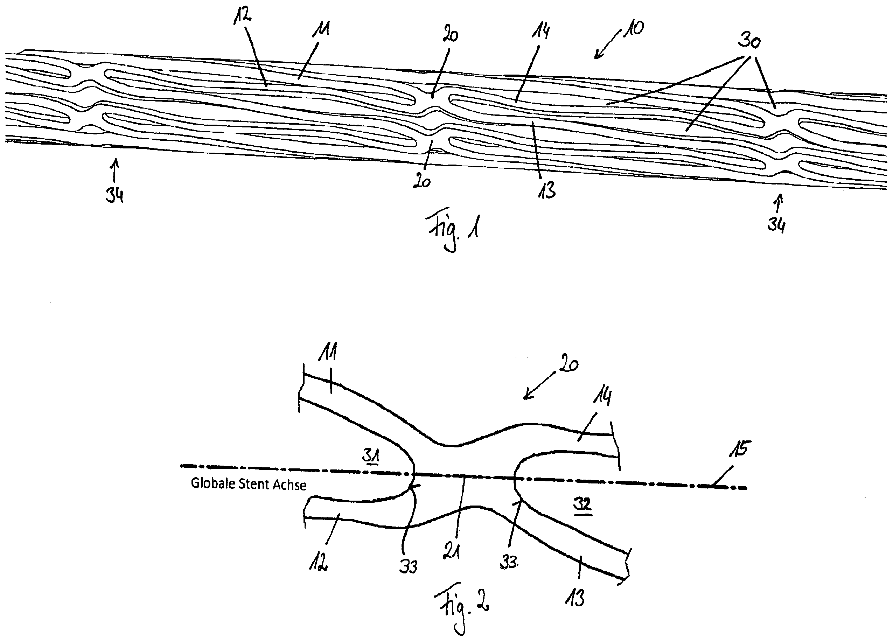

Fig. 1 zeigt die Gitterstruktur 10 im Zuführzustand. Es ist gut erkennbar, dass die Stege 11, 12, 13, 14 in Umfangsrichtung teilweise aneinander liegen, so dass eine weitere Komprimierung blockiert ist. Mit anderen Worten begrenzt die Stegbreite der einzelnen Stege 11, 12, 13, 14 die Komprimierbarkeit der Gitterstruktur 10. Ferner ist erkennbar, dass die Stegverbinder 20 im Wesentlichen jeweils auf einer gemeinsamen Umfangslinie angeordnet sind. Insgesamt bilden also mehrere Zellen 30 in Umfangrichtung der Gitterstruktur 10 einen Zellenring 34. Mehrere in Längsrichtung miteinander verbundene Zellenringe 34 bilden die gesamte Gitterstruktur 10.

In diesem Zusammenhang wird darauf hingewiesen, dass die Gitterstruktur 10 lediglich abschnittsweise aus miteinander verbundenen Zellenringen gebildet sein kann, die denselben Querschnittsdurchmesser aufweisen. Es ist vielmehr auch möglich, dass die Gitterstruktur 10 abschnittsweise eine von einer Zylinderform verschiedene Geometrie aufweist. Beispielsweise kann die Gitterstruktur zumindest an einem proximalen Ende trichterförmig ausgebildet sein. Eine derartige Konfiguration ist bei medizinischen Vorrichtungen vorteilhaft, die als Thrombenfänger bzw. allgemein als Thrombektomiedevice, eingesetzt werden. Die Gitterstruktur 10 kann in derartigen Fällen im Wesentlichen eine korbähnliche Struktur bilden. Gitterstrukturen 10, die vollständig zylinderförmig ausgebildet sind, werden bei medizinischen Vorrichtungen eingesetzt, die einen Stent bilden. Stents können zur Stützung von Blutgefäßen bzw. allgemein Körperhohlorganen und/oder zur Abdeckung von Aneurysmen genutzt werden.

In Fig. 2 ist ein Stegverbinder 20 der Gitterstruktur 10 gemäß Fig. 1 im Detail gezeigt. Der Stegverbinder 20 verbindet vier Stege 11, 12, 13, 14 miteinander. Der Einfachheit halber werden im Rahmen der Anmeldung die an einem Stegverbinder 20 zusammengeführten Stege 11, 12, 13, 14 im Gegenuhrzeigersinn durchnummeriert, um eine klare Zuordnung zu schaffen. Dabei ist vorgesehen, dass ein erster Steg 11 und ein zweiter Steg 12 einer ersten Zelle 30 und ein dritter Steg 13 und ein vierter Steg 14 einer zweiten Zelle 30 zugeordnet sind, wobei die Zellen 30 in Längsrichtung der Gitterstruktur 10 unmittelbar benachbart sind. Mit anderen Worten trennt der Stegverbinder 20 zwei in Längsrichtung benachbart zueinander angeordnete Zellen 30 der Gitterstruktur 10 voneinander. Im Bereich des Stegverbinders 20 bilden die Zellen 30 jeweils Zellenspitzen 31, 32. Eine erste Zellenspitze 31 ist durch ersten Steg 11 und den zweiten Steg 12 begrenzt. Eine zweite Zellenspitze 32 ist durch den dritten Steg 13 und den vierten Steg 14 begrenzt.

Die Zellenspitzen 31, 32 bilden bzw. umfassen jeweils eine Krümmung 33. Die Krümmung 33 ergibt sich durch den Übergang der Seitenflächen von in Umfangsrichtung benachbarten Stegen 11, 12, 13, 14. Konkret bildet die erste Zellenspitze 31 eine Krümmung 33, die sich aus dem Übergang des ersten Stegs 11 zum zweiten Steg 12 im Bereich des Stegverbinders 20 ergibt. Entsprechend ist die Krümmung 33 der zweiten Zellenspitze 32 durch einen gekrümmten Übergang zwischen dem dritten Steg 13 und dem vierten Steg 14 am Stegverbinder 20 geformt. Die Krümmungen 33 weisen vorzugsweise eine parabelartige Form auf. Jede der Krümmungen 33 weist ein Maximum bzw. einen Scheitelpunkt auf, also einen Punkt, an welchem der kleinste Krümmungsradius vorliegt. Die Entfernung zwischen den Scheitelpunkten der an einem Stegverbinder 20 gegenüberliegenden Krümmungen 33 entspricht dem kürzesten Abstand zwischen den Zellenspitzen 31, 32. Die Verbindungslinie, d.h. der Abstand, zwischen den beiden Maxima bzw. Scheitelpunkten der Krümmungen 33 bildet die Verbinderachse 21. Mit anderen Worten enspricht die Verbinderachse 21 dem kürzesten Abstand zwischen den beiden Krümmungen 33 der ersten Zellenspitze 31 und der zweiten Zellenspitze 32.

Die Verbinderachse 21 kennzeichnet die Ausrichtung des Stegverbinders 20 im Bezug auf unterschiedliche Expansionsgrade. Im Allgemeinen ändert sich der Winkel zwischen der Verbinderachse 21 und der Längsachse 15 der Gitterstruktur 10, wenn die Gitterstruktur 10 komprimiert oder expandiert wird. Im Zuführzustand ist die Verbinderachse 21 vorzugsweise parallel zur Längsachse 15 der Gitterstruktur 10 ausgerichtet. Es ist allerdings auch möglich, dass die Verbinderachse 21 im Zuführzustand unter einem Winkel zur Längsachse 15 der Gitterstruktur 10 angeordnet ist. Der Winkel beträgt vorzugsweise höchstens 5°, insbesondere höchstens 4°, insbesondere höchstens 3°, insbesondere höchstens 2°, insbesondere höchstens 1°.

Die Längsachse 15 der Gitterstruktur 10 ist in den Figuren durch eine strichpunktierte Linie mit der Bezeichnung "global stent-axis" dargestellt. In Fig. 2 ist erkennbar, dass die Zellenspitzen 31, 32 im Zuführzustand im Wesentlichen in Längsrichtung gegenüberliegend angeordnet sind, insbesondere ohne einen Versatz in Umfangsrichtung. In dieser Konfiguration weist die Gitterstruktur 10 eine besonders hohe axiale Stabilität auf. Dies ist zur Zuführung der Gitterstruktur 10 durch einen Katheter vorteilhaft. Insbesondere stützen sich die Zellenspitzen 31, 32 in längsaxialer Richtung, also parallel zur Längsachse 15, gegenseitig ab.

Bei der Entlassung der Gitterstruktur 10 aus einem Katheter bzw. allgemein einem Zuführsystem weitet sich die Gitterstruktur 10 selbsttätig radial aus. Dabei durchläuft die Gitterstruktur 10 mehrere Expansionsgrade, bis die Gitterstruktur 10 den implantierten Zustand erreicht. Im implantierten Zustand übt die Gitterstruktur 10 vorzugsweise eine Radialkraft auf umliegende Gefäßwände aus. Der implantierte Zustand entspricht vorzugsweise einem Expansionsgrad der Gitterstruktur 10, der kleiner 100% und größer als 10% ist. Der implantierte Zustand wird auch als "intended use-configuration" bezeichnet.

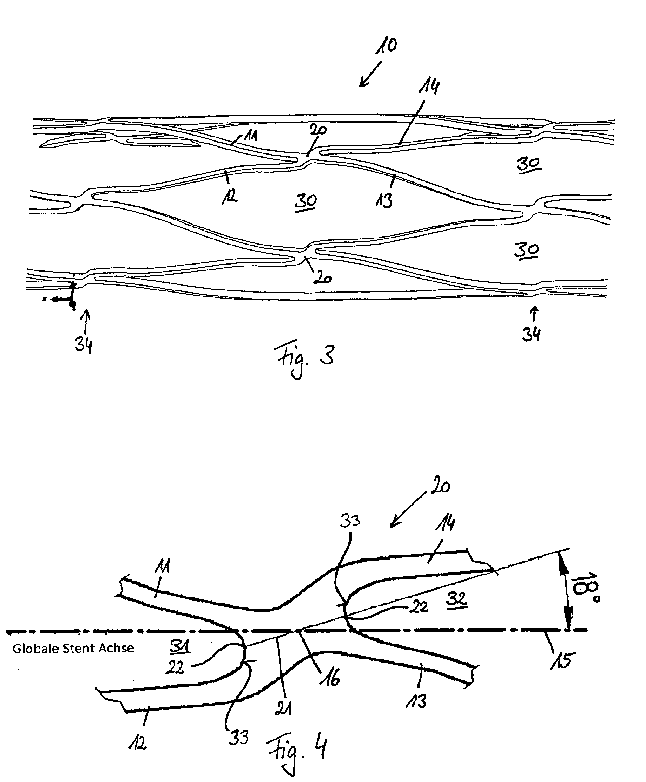

In den Figuren 3-8 ist die Gitterstruktur 10 bzw. der Stegverbinder 20 bei unterschiedlichen Expansionsgraden gezeigt. So zeigt Fig. 3 beispielhaft eine Seitenansicht der Gitterstruktur 10 bei einem Expansionsgrad von 44,4%. Es ist gut erkennbar, dass die Stegverbinder 20 trotz der radialen Expansion der Gitterstruktur 10 weiterhin auf einer gemeinsamen Umfangslinie angeordnet sind. Insbesondere findet bei der Expansion der Gitterstruktur 10 kein längsaxialer Versatz der einzelnen Stegverbinder 20 zueinander statt. Die Zellen 30 weiten sich erkennbar aus, wobei sich die in Umfangsrichtung benachbarten Stege 11, 12, 13, 14 V-förmig aufklappen. Bei der Expansion der Gitterstruktur 10 vergrößert sich also die Zellenhöhe, die in Umfangsrichtung der Gitterstruktur 10 bemessen ist. Gleichzeitig erfolgt aufgrund des Foreshorteningeffekts eine Reduktion der Zellenbreite, wobei die Breite der Zelle 30 in Längsrichtung der Gitterstruktur 10 ermittelt wird.

Fig. 4 zeigt den Stegverbinder 20 der Gitterstruktur 10 bei einem Expansionsgrad der Gitterstruktur 10 von 44,4%. Insofern stellt Fig. 4 eine Detailansicht aus Fig. 3 dar. Es ist gut erkennbar, dass die Zellenspitzen 31, 32 durch die Expansion der Gitterstruktur 10 in Umfangsrichtung zueinander versetzt werden. Insbesondere dreht bzw. rotiert der Stegverbinder 20 bei der Expansion der Gitterstruktur 10, so dass sich zwischen der Verbinderachse 21 und der Längsachse 15 der Gitterstruktur 10 ein Winkelversatz bildet. Bei einem Expansionsgrad von 44,4% beträgt der Winkelversatz vorzugsweise etwa 18°. Konkret ist zwischen der Verbinderachse 21 und der Längsachse 15 der Gitterstruktur 10 bei einem Expansionsgrad von 44,4% ein Winkel gebildet, der einen Wert von 18° einnimmt. Die Rotation des Stegverbinders 20 erfolgt beim Übergang der Gitterstruktur 10 vom Zuführzustand bis zu einem Expansionsgrad von 44,4% bzw. allgemein von weniger als 50% im Wesentlichen im Gegenuhrzeigersinn.

In Fig. 4 ist ebenfalls erkennbar, dass die Verbinderachse 21 die Längsachse 15 der Gitterstruktur 10 schneidet. Der Schnittpunkt zwischen der Verbinderachse 21 und der Längsachse 15 der Gitterstruktur 10 wird als Rotationspunkt 16 bezeichnet. Der Rotationspunkt 16 bezeichnet den Punkt im Stegverbinder 20, um welchen der Stegverbinder 20 rotiert. Der Stegverbinder 20 dreht also um den Rotationspunkt 16 während der Expansion der Gitterstruktur 10, wobei sich die Rotationsrichtung ändern kann.

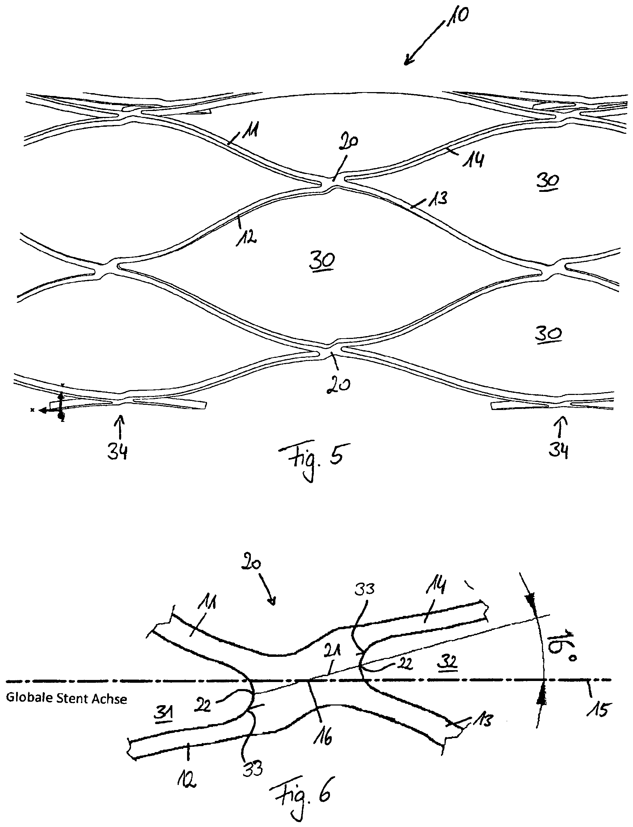

Fig. 5 zeigt einen weiteren Zwischenzustand der Gitterstruktur 10, insbesondere bei einem Expansionsgrad von 66,7%. Auch in diesem Zustand liegen die Stegverbinder 20 weiterhin auf einer gemeinsamen Umfangslinie. Die Zellen 30 sind gegenüber dem Zwischenzustand gemäß Fig. 4 mit einer größeren Höhe und einer kleineren Breite erkennbar.

In der Detailansicht gemäß Fig. 6 ist der Stegverbinder 20 bei einem Expansionsgrad der Gitterstruktur 10 von 66,7% gezeigt. Der Stegverbinder 20 weist eine Verbinderachse 21 auf, die in einem Winkel von 16° zur Längsachse 15 der Gitterstruktur 10 angeordnet ist. Im Allgemeinen ist vorgesehen, dass bei einem Expansionsgrad von größer als 50% der Winkel zwischen der Verbinderachse 21 und der Längsachse 15 der Gitterstruktur 10 kleiner als bei einem Expansionsgrad der Gitterstruktur 10 von weniger als 50% ist. Mit anderen Worten ändert sich die Rotationsrichtung des Stegverbinders 20 während der Expansion der Gitterstruktur 10. Der Wechsel der Rotationsrichtung erfolgt vorzugsweise bei etwa 50% Expansionsgrad, insbesondere bei einem Expansionsgrad zwischen 44% und 45%. Der Stegverbinder 20 dreht während der Expansion der Gitterstruktur 10 zunächst im Gegenuhrzeigersinn, wechselt dann die Rotationsrichtung und dreht anschließend im Uhrzeigersinn zurück.

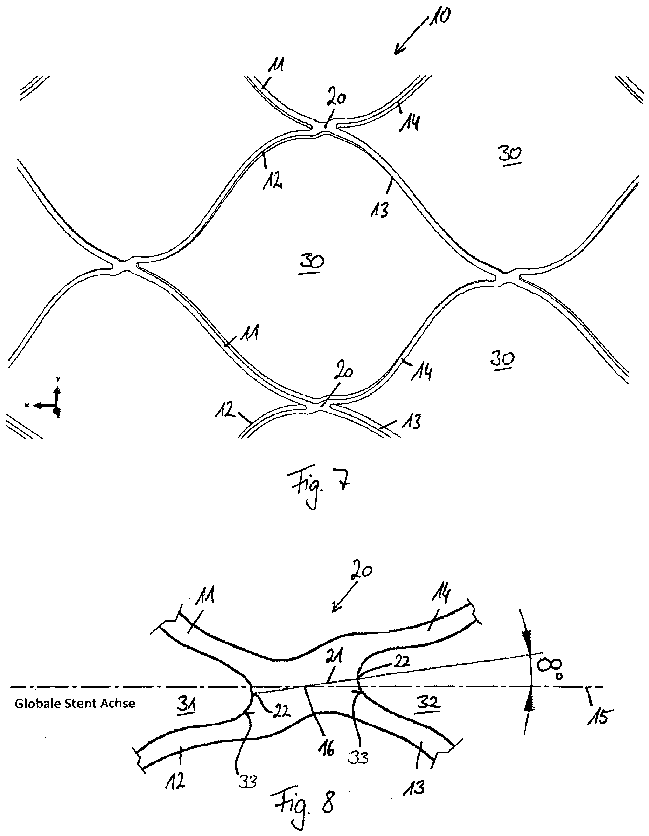

Bei einem Expansionsgrad von 88,9%, also einem Zwischenzustand, wie er den in Figuren 7 und 8 dargestellt ist, weist der Stegverbinder 20 zur Längsachse 15 der Gitterstruktur 10 einen Winkel auf, der kleiner als bei einem Expansionsgrad von 44,4% und 66,7% ist. Wie in Fig. 8 gezeigt ist, beträgt der Winkelversatz zwischen der Verbinderachse 21 und der Längsachse 15 der Gitterstruktur 10 bei einem Expansionsgrad von 88,9% etwa 8°. Mit anderen Worten reduziert sich der Versatz zwischen den Zellenspitzen 31, 32 in Umfangsrichtung. Fig. 7 lässt gut erkennen, dass bei einem Zwischenzustand, der im Wesentlichen einem Expansionsgrad von etwa 90% entspricht, die Zellenhöhe fast an die Zellenbreite der Zelle 30 angepasst ist. Die Stegverbinder 20 der Gitterstruktur 10 sind weiterhin auf einer gemeinsamen Umfangslinie angeordnet. Dasselbe gilt für die Stegverbinder 20 in Längsrichtung der Gitterstruktur 10.

Im Allgemeinen ändern die Stegverbinder 20 der Gitterstruktur 10 ihre Ausrichtung relativ zueinander sowohl in Umfangsrichtung, als auch in Längsrichtung bei der Expansion der Gitterstruktur 10 nicht. Vielmehr ändert sich die Ausrichtung der Verbinderachse 21 jedes einzelnen Stegverbinders 20. Mit anderen Worten drehen die Stegverbinder 20 bei der Expansion der Gitterstruktur 10, behalten Ihre Relativposition mit Bezug auf in Längsrichtung und Umfangsrichtung benachbarten Stegverbinder 20 jedoch im Wesentlichen bei. Lediglich der Abstand zwischen den Stegverbindern 20 ändert sich, wobei sich die Stegverbinder 20 auf geradlinigen Bewegungslinien voneinander entfernen oder, bei Komprimierung der Gitterstruktur 10, einander annähern.

In Fig. 9 ist eine Seitenansicht der Gitterstruktur 10 gezeigt, wobei die Gitterstruktur 10 im Herstellzustand vorliegt. Mit anderen Worten ist die Gitterstruktur 10 vollständig expandiert. Die Gitterstruktur 10 weist im Wesentlichen einen radialkraftfreien Zustand auf. Die Zelle 30 weist in diesem Zustand eine Zellenhöhe auf, die im Wesentlichen der Zellenbreite entspricht. Die Grundform der Zelle ist in diesem Fall im Wesentlichen quadratisch, entspricht also einer gleichwinkligen bzw. rechtwinkligen Raute. Zwar verlaufen die Stege 11, 12, 13, 14 der Zelle 30 nicht geradlinig. Verbindet man jedoch die Zellenspitzen 31, 32 der Zelle geradlinig miteinander, so zeigt sich im Wesentlichen eine quadratische Grundform.

In Fig. 10 ist der Stegverbinder 20 im Detail dargestellt, wenn die Gitterstruktur 10 im Herstellzustand gemäß Fig. 9 vorliegt. Durch die sich umkehrende Rotation des Stegverbinders 20 während der Expansion der Gitterstruktur 10, nimmt der Stegverbinder 20 im Herstellzustand wieder die ursprüngliche Ausrichtung ein. Insbesondere ist die Verbinderachse 21 im Herstellzustand parallel zur Längsachse 15 der Gitterstruktur 10 ausgerichtet. Die Zellenspitzen 31, 32 sind also in Längsrichtung der Gitterstruktur 10 gegenüberliegend am Stegverbinder 20 angeordnet. Mit anderen Worten fluchten die Zellenspitzen 31, 32 zueinander. Es ist allerdings auch möglich, dass die Verbinderachse 21 im Herstellzustand unter einem Winkel zur Längsachse 15 der Gitterstruktur 10 angeordnet ist. Der Winkel beträgt vorzugsweise höchstens 5°, insbesondere höchstens 4°, insbesondere höchstens 3°, insbesondere höchstens 2°, insbesondere höchstens 1°.

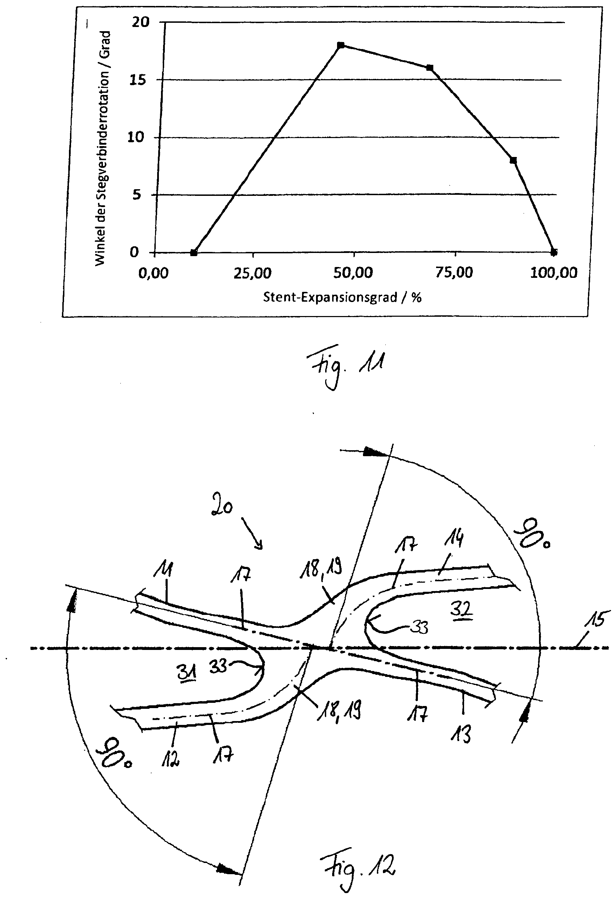

Fig. 11 zeigt diagrammartig den Verlauf der Stegverbinderrotation über die Expansion der Gitterstruktur 10. Beginnend bei einem Zuführzustand, der einem Expansionsgrad von etwa 10% entspricht, erhöht sich der Winkel zwischen Verbinderachse 21 und Längsachse 15 der Gitterstruktur 10 zunächst. Insbesondere steigt der Rotationswinkel auf einen Wert von bis zu etwa 18° an. Dieser Wert wird etwa bei einem Expansionsgrad von 44% bis 45% erreicht. Anschließend ändert sich die Drehrichtung des Stegverbinders 20, so dass der Winkel zwischen der Verbinderachse 21 und der Längsachse 15 der Gitterstruktur 10 sinkt. Insbesondere reduziert sich der Winkel zwischen Verbinderachse 21 und Längsachse 15 der Gitterstruktur 10 bis zum vollständig expandierten Zustand bzw. zum Herstellzustand der Gitterstruktur 10. Im Herstellzustand, also bei einem Expansionsgrad von 100%, beträgt der Winkel zwischen der Verbinderachse 21 und der Längsachse 15 der Gitterstruktur 10 0%. Die Verbinderachse 21 ist also parallel zur Längsachse 15 der Gitterstruktur 10 angeordnet. Im Allgemeinen ist die Verbinderachse 21 des Stegverbinders 20 sowohl im Zuführzustand, als auch im Herstellzustand der Gitterstruktur 10 parallel zur Längsachse 15 der Gitterstruktur 10 ausgerichtet. In den Zwischenzuständen zwischen dem Zuführzustand und dem Herstellzustand zeigt sich ein Winkel zwischen der Verbinderachse 21 und der Längsachse 15 der Gitterstruktur 10.

Fig. 12 zeigt beispielhaft einen Stegverbinder 20 der Gitterstruktur 10 bei einem Expansionsgrad der Gitterstruktur 10 von 44,4%. In Fig. 12 sind ferner die neutralen Fasern 17 der einzelnen Stege 11, 12, 13, 14 durch strichpunktierte Linien eingezeichnet. Eine weitere strichpunktierte Linie, die horizontal in der Bildebene verläuft, stellt die Lage der Längsachse 15 der Gitterstruktur 10 dar.

Am Stegverbinder 20 treffen insgesamt vier Stege 11, 12, 13, 14 aufeinander. Dabei unterscheiden sich die Stege 11, 12, 13, 14 einerseits durch ihre Formgebung und andererseits durch ihre Stegbreite. Insbesondere sind ein erster Steg 11 und dritter Steg 13 mit einer Stegbreite versehen, die kleiner als die Stegbreite des zweiten Stegs 12 und des vierten Stegs 14 ist. Dabei wird die durchschnittliche Stegbreite betrachtet. Ferner ist erkennbar, dass der erste Steg 11 und der dritte Steg 13 im Wesentlichen geradlinig zueinander fluchtend in den Stegverbinder 20 münden. Konkret weisen der erste Steg 11 und der dritte Steg 13 jeweils eine neutrale Faser 17 auf, wobei die neutralen Fasern 17 des ersten Stegs 11 und des dritten Stegs 13 fluchtend zueinander ausgerichtet sind. Die neutralen Fasern 17 des ersten Stegs und des dritten Stegs 13 gehen also im Wesentlichen geradlinig ineinander über, so dass sich eine gemeinsame neutrale Faser 17 bildet, die den Stegverbinder 20 durchkreuzt.

Der zweite Steg 12 und der vierte Steg 14 weisen eine von dem ersten Steg 11 und dem dritten Steg 13 verschiedene Form auf. Kennzeichnend für den zweiten Steg 12 und den vierten Steg 14 ist ein gekrümmter Verlauf im Bereich des Stegverbinders 20. Konkret weisen der zweite Steg 12 und der vierte Steg 14 jeweils einen abgewinkelten Endabschnitt 19 auf. Die abgewinkelten Endabschnitte 19 bilden jeweils eine Wurzel 18 des zweiten Stegs 12 und des vierten Stegs 14. Konkret weisen der zweite Steg 12 und der vierte Steg 14 jeweils eine verbreitere Wurzel 18 auf.

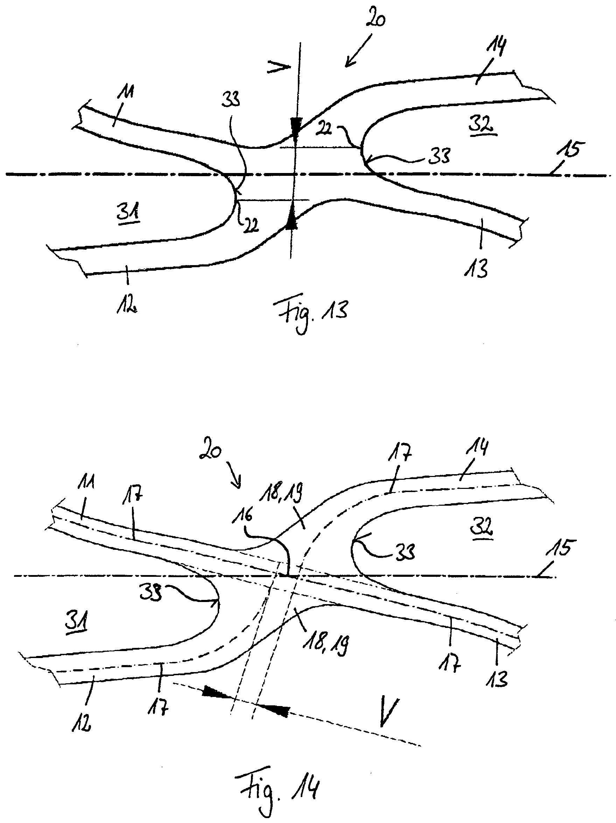

Die diametral gegenüberliegenden Stege 12, 14, die als zweiter Steg 12 und als vierter Steg 14 bezeichnet sind, münden im Wesentlichen versetzt zueinander in den Stegverbinder 20. Dies ist deutlich durch den Verlauf der neutralen Fasern 17 der Stege 11, 12, 13, 14 erkennbar. Sowohl der zweite Steg 12, als auch der vierte Steg 14 weisen jeweils eine neutrale Faser 17 auf, die unter einem Winkel auf die neutrale Faser 17, insbesondere die gemeinsame neutrale Faser 17, des ersten Stegs 11 und des dritten Stegs 13 treffen. Vorzugsweise beträgt der Winkel zwischen der neutralen Faser 17 des zweiten Stegs 12 und der gemeinsamen neutralen Faser 17 des ersten Stegs und des dritten Stegs 13 90°. Dasselbe gilt für die neutrale Faser 17 des vierten Stegs 14, die ebenfalls unter einem Winkel von vorzugsweise 90° auf die gemeinsame neutrale Faser 17 des ersten Stegs 11 und des dritten Stegs 13 trifft. Die neutralen Fasern 17 des zweiten Stegs 12 und des vierten Stegs 14 sind hingegen versetzt zueinander angeordnet. Fig. 14 zeigt den Stegverbinder 20 aus Fig. 12, wobei der Faserversatz V zwischen den neutralen Fasern 17 des zweiten Stegs 12 und des vierten Stegs 14 verdeutlicht ist.

Zwar ist grundsätzlich ein Winkel zwischen den neutralen Fasern des zweiten Stegs und des vierten Stegs in Bezug auf die gemeinsame neutrale Faser 17 des ersten Stegs 11 und des dritten Stegs 13 von 90°, also eine senkrechte Ausrichtung, bevorzugt. Es sind jedoch andere Winkel möglich. Beispielsweise können die neutralen Fasern des zweiten Stegs 12 und des vierten Stegs 14 auch unter einem Winkel von weniger als 90°, insbesondere höchstens 75°, insbesondere höchstens 55°, insbesondere höchstens 50°, insbesondere höchstens 45°, auf die gemeinsame neutrale Faser 17 des ersten Stegs 11 und des dritten Stegs 13 treffen.

Im Allgemeinen ist aus Fig. 12 erkennbar, dass der erste Steg 11 und der dritte Steg 13 im Wesentlichen fluchtend bzw. linientreu zueinander angeordnet sind. Der zweite Steg 12 und der vierte Steg 14 weisen hingegen im Bereich des Stegverbinders 20 einen S-förmigen Verlauf auf. Dabei bilden die abgewinkelten Endabschnitte 19 des zweiten Stegs 12 und des vierten Stegs 14 einen durch den Stegverbinder 20 gebildeten Zwischenabschnitt zwischen zwei gegenläufigen Biegungen des S-förmigen Verlaufs der beiden diametral gegenüberliegend angeordneten Stege 12, 14, die als zweiter Steg 12 und als vierter Steg 14 bezeichnet sind.

Fig. 13 zeigt zur Verdeutlichung den Stegverbinder gemäß Fig. 12, wobei der in Umfangsrichtung gebildete Versatz der Zellenspitzen 31, 32 zueinander verdeutlicht ist. Der Stegverbinder 20 gemäß Fig. 13 weist eine Verbinderachse 21 auf, die in einem Winkelversatz zur Längsachse 15 der Gitterstruktur 10 angeordnet ist. Der Winkelversatz beträgt vorzugsweise etwa 18°. Mit anderen Worten zeigt Fig. 13 den Stegverbinder 20 bei einem Expansionsgrad der Gitterstruktur 10 von etwa 44,4%. Zwischen den Endpunkten 22 der Verbinderachse 21 besteht in diesem Zwischenzustand der Gitterstruktur 10 ein Umfangsversatz V, der durch entsprechende Pfeile in Fig. 13 dargestellt ist. Der Umfangsversatz V beträgt im Verhältnis zur Stegbreite des zweiten Stegs 12 und des vierten Stegs 14 vorzugsweise wenigstens 0,5, insbesondere wenigstens 1,0, insbesondere wenigstens 1,5, insbesondere wenigstens 2,0. Bei dem Ausführungsbeispiel gemäß Fig. 13 beträgt der Umfangsversatz V im Verhältnis zur Stegbreite des zweiten Stegs 12 und des vierten Stegs 14 1,0. Mit anderen Worten entspricht der Umfangsversatz der Zellenspitzen 31, 32 bzw. der Endpunkte 22 der Verbinderachse 21 der Stegbreite des zweiten Stegs 12 bzw. des vierten Stegs 14. Der Umfangsversatz V ist abhängig vom Winkelversatz. Insofern beträgt der Umfangsversatz V im Zuführzustand und/oder im Herstellzustand der Gitterstruktur 10 vorzugsweise Null.

Grundsätzlich ist im Rahmen der Erfindung vorgesehen, dass die Rotation des Stegverbinders 20 höchstens 30°, insbesondere höchstens 20°, insbesondere höchstens 18°, insbesondere höchstens 15°, insbesondere höchstens 10°, insbesondere höchstens 5°, betragen kann. In den dargestellten Ausführungsbeispielen beträgt der Winkelversatz zwischen der Verbinderachse 21 und der Längsachse 15 der Gitterstruktur 10 maximal 18°, insbesondere bei einem Expansionsgrad der Gitterstruktur 10 von 44,4%.

Um eine hohe Flexibilität bereitzustellen, ist es vorteilhaft, wenn die Stege 11, 12, 13, 14 unterschiedliche Stegbreiten aufweisen. So können diametral gegenüberliegende Stege, insbesondere der erste Steg 11 und der dritte Steg 13, eine erste Stegbreite und kreuzend angeordnete, diametral gegenüberliegende Stege, insbesondere der zweite Steg 12 und der vierte Steg 14 eine zweite Stegbreite aufweisen. Das Verhältnis zwischen der ersten Stegbreite und der zweiten Stegbreite, d.h. das Stegbreitenverhältnis, kann wenigstens 1:1,25, insbesondere wenigstens 1:1,5, insbesondere wenigstens 1:1,75, insbesondere wenigstens 1:2, betragen.

Als besonders geeignet hat sich ein Stegbreitenverhältnis von 1:1,2 oder 1:1,3 herausgestellt, insbesondere für eine Gitterstruktur 10, die in Umfangsrichtung sechs Zellen 30 aufweist. Bei einer Gitterstruktur 10, die mehr als zwölf in Umfangsrichtung unmittelbar benachbart angeordnete Zellen 30 aufweist, beträgt das Stegbreitenverhältnis vorzugsweise 1:1,2 bzw. 1:1,25.

Grundsätzlich wird die Flexibilität der Gitterstruktur 10 erhöht, wenn ein relativ kleines Stegbreitenverhältnis gewählt ist. Bei einem Stegbreitenverhältnis von 1:1,25 (Stegbreite des ersten Stegs 11 bzw. dritten Stegs 13 zur Stegbreite des zweiten Stegs 12 bzw. vierten Stegs 14) hat sich eine besonders hohe Flexibilität gezeigt, da dies zu einer verbesserten Rotation des Stegverbinders 20 führt. Gleichzeitig wird bei einem derartigen Stegbreitenverhältnis eine ausreichende Expansionskraft bereitgestellt.

Ferner ist im Allgemeinen vorgesehen, dass die Gitterstruktur 10 in Umfangsrichtung höchstens 48, insbesondere höchstens 24, insbesondere höchstens 16, insbesondere höchstens 12, insbesondere höchstens 6, Zellen 30 aufweist. Mit anderen Worten kann jeder Zellenring 34 der Gitterstruktur 10 höchstens aus 48, insbesondere höchstens aus 24, insbesondere höchstens aus 16, insbesondere höchstens aus 12, insbesondere höchstens aus 6, Zellen gebildet sein.

In einem konkreten Ausführungsbeispiel bildet die medizinische Vorrichtung einen Stent mit einer Gitterstruktur 10. Die Gitterstruktur 10 bzw. der Stent kann einen Außendurchmesser im Herstellzustand von 3,5 mm oder 4,5 mm aufweisen. Die axiale Länge des Stents bzw. der Gitterstruktur 10 beträgt vorzugsweise 15 mm, 20 mm oder 35 mm. Das Stegbreitenverhältnis beträgt vorzugsweise 1:1,25, wobei die erste Stegbreite 40 µm und die zweite Stegbreite 32µm umfasst. Die Gitterstruktur 10 ist vorzugsweise als sechs-zellige Gitterstruktur 10 ausgebildet, d.h. die Gitterstruktur 10 weist Zellenringe 34 aus jeweils sechs Zellen 30 auf.

Grundsätzlich eignet sich die Erfindung nicht nur zum Einsatz als Stent, sondern auch als Clot-Retriever oder Flowdiverter, insbesondere mit einer lasergeschnittenen Gitterstruktur 10.

### Bezugszeichenliste

- 10: Gitterstruktur
- 11: erster Steg
- 12: zweiter Steg
- 13: dritter Steg
- 14: vierter Steg
- 15: Längsachse
- 16: Rotationspunkt
- 17: neutrale Faser
- 18: Wurzel
- 19: abgewinkelter Endabschnitt
- 20: Stegverbinder
- 21: Verbinderachse
- 22: Endpunkt
- 30: Zelle
- 31: erste Zellenspitze
- 32: zweite Zellenspitze
- 33: Krümmung
- 34: Zellenring

## Patentansprüche

1. Medizinische Vorrichtung zur Einfuhr in ein Körperhohlorgan mit einer komprimierbaren und expandierbaren Gitterstruktur (10) aus Stegen (11, 12, 13, 14), die durch Stegverbinder (20) einstückig miteinander verbunden sind und geschlossene Zellen (30) der Gitterstruktur (10) begrenzen, wobei die Zellen (30) durch jeweils vier Stege (11, 12, 13, 14) begrenzt sind und im Herstellzustand eine rautenförmige Grundform aufweisen, wobei die Stegverbinder Kreuzungsstellen der Stege (11, 12, 13, 14) bilden und jeweils eine Verbinderachse (21) aufweisen, die sich zwischen zwei in Längsrichtung der Gitterstruktur (10) benachbarten Zellen (30) erstreckt, und wobei die Gitterstruktur (10) selbstexpandierbar ist,
**dadurch gekennzeichnet, dass**
die Stegverbinder (20) beim Übergang der Gitterstruktur (10) vom Herstellzustand in einen komprimierten Zustand derart rotieren, dass sich ein Winkel zwischen der Verbinderachse (21) und einer Längsachse (15) der Gitterstruktur (10) beim Übergang der Gitterstruktur (10) von einem vollständig expandierten Herstellzustand in einen teilexpandierten Zwischenzustand ändert, insbesondere erhöht.

2. Medizinische Vorrichtung nach Anspruch 1,
**dadurch gekennzeichnet, dass**
der Winkel zwischen der Verbinderachse (21) und der Längsachse (15) im Herstellzustand und/oder in einem komprimierten Zuführzustand der Gitterstruktur (10) höchstens 5°, insbesondere höchstens 4°, insbesondere höchstens 3°, insbesondere höchstens 2°, insbesondere höchstens 1°, beträgt.

3. Medizinische Vorrichtung nach Anspruch 1 oder 2,
**dadurch gekennzeichnet, dass**
die Verbinderachse (21) im Herstellzustand und/oder in einem komprimierten Zuführzustand der Gitterstruktur (10) parallel zur Längsachse (15) der Gitterstruktur (10) ausgerichtet ist.

4. Medizinische Vorrichtung nach einem der vorhergehenden Ansprüche,
**dadurch gekennzeichnet, dass**
der Stegverbinder (20) beim Übergang der Gitterstruktur (10) vom Herstellzustand in den komprimierten Zustand, insbesondere den Zuführzustand, die Rotationsrichtung wechselt.

5. Medizinische Vorrichtung nach einem der vorhergehenden Ansprüche,
**dadurch gekennzeichnet, dass**
die Verbinderachse (21) dem kürzesten Abstand zwischen zwei Zellenspitzen (31, 32) von in Längsrichtung am Stegverbinder (20) gegenüberliegenden Zellen (30) entspricht.

6. Medizinische Vorrichtung nach einem der vorhergehenden Ansprüche,
**dadurch gekennzeichnet, dass**
in Längsrichtung benachbarte Zellen (30) der Gitterstruktur (10) jeweils eine Zellenspitze (31, 32) mit einer Krümmung (33) umfassen, wobei die Krümmung (33) ein Maximum, insbesondere einen Scheitelpunkt, aufweist, das einen Endpunkt (22) der Verbinderachse (21) bildet.

7. Medizinische Vorrichtung nach einem der vorhergehenden Ansprüche,
**dadurch gekennzeichnet, dass**
die Verbinderachse (21) zumindest in einem teilexpandierten Zustand der Gitterstruktur (10) die Längsachse (15) der Gitterstruktur (10) in einem Schnittpunkt schneidet, der einen Rotationspunkt (16) bildet, um welchen der Stegverbinder (20) rotiert.

8. Medizinische Vorrichtung nach Anspruch 7,
**dadurch gekennzeichnet, dass**
der Stegverbinder (20) bezogen auf den Rotationspunkt (16) punktsymmetrisch ausgebildet ist.

9. Medizinische Vorrichtung nach einem der vorhergehenden Ansprüche,
**dadurch gekennzeichnet, dass**
jeweils vier Stege (11, 12, 13, 14) an einem Stegverbinder (20) angeordnet sind, wobei jeweils ein erster und dritter Steg (11, 13) und ein zweiter und vierter Steg (12, 14) diametral gegenüberliegend angeordnet sind.

10. Medizinische Vorrichtung nach Anspruch 9,
**dadurch gekennzeichnet, dass**
jeweils eine neutrale Faser (17) des ersten und dritten Stegs (11, 13) senkrecht auf den neutralen Fasern (17) des zweiten und vierten Stegs (12, 14) steht.

11. Medizinische Vorrichtung nach Anspruch 10,
**dadurch gekennzeichnet, dass**
die neutralen Fasern (17) des zweiten und vierten Stegs (12, 14) fluchtend zueinander und die neutralen Fasern (17) des ersten und dritten Stegs (11, 13) versetzt zueinander angeordnet sind.

12. Medizinische Vorrichtung nach einem der Ansprüche 9 bis 11,
**dadurch gekennzeichnet, dass**
der erste und dritte Steg (11, 13) jeweils eine verbreiterte Wurzel (18) aufweisen.

13. Medizinische Vorrichtung nach einem der Ansprüche 9 bis 12,
**dadurch gekennzeichnet, dass**
der erste und dritte Steg (11, 13) im Bereich des Stegverbinders (20) einen S-förmigen Verlauf und/oder der zweite und vierte Steg (12, 14), insbesondere deren neutrale Fasern (17), im Wesentlichen einen geradlinigen Verlauf aufweisen.

14. Medizinische Vorrichtung nach einem der vorhergehenden Ansprüche,
**dadurch gekennzeichnet, dass**
der Stegverbinder (20) eine taillierte Form aufweist.

15. Medizinische Vorrichtung nach einem der vorhergehenden Ansprüche,
**dadurch gekennzeichnet, dass**
die Verbinderachse (21) bei einem Expansionsgrad der Gitterstruktur (10) von 5% bis 15%, insbesondere von 8% bis 12%, insbesondere 10% oder 9.9%, und/oder bei einem Expansionsgrad von mindestens 90%, insbesondere mindestens 95%, insbesondere mindestens 98%, insbesondere 100%, jeweils unter einem Winkel von höchstens 5°, insbesondere höchstens 4°, insbesondere höchstens 3°, insbesondere höchstens 2°, insbesondere höchstens 1°, vorzugsweise parallel, zur Längsachse (15) der Gitterstruktur (10) ausgerichtet ist.

16. Medizinische Vorrichtung nach einem der vorhergehenden Ansprüche,
**dadurch gekennzeichnet, dass**
die Verbinderachse (21) bei einem Expansionsgrad von 40% bis 50%, insbesondere von 42% bis 48%, insbesondere 44,4%, mit der Längsachse (15) der Gitterstruktur (10) einen Winkel von 15° bis 20°, insbesondere von 16° bis 19°, insbesondere 18°, einschließt.

17. Medizinische Vorrichtung nach einem der vorhergehenden Ansprüche,
**dadurch gekennzeichnet, dass**
die Verbinderachse (21) bei einem Expansionsgrad von 60% bis 70%, insbesondere von 62% bis 68%, insbesondere 66,7%, mit der Längsachse (15) der Gitterstruktur (10) einen Winkel von 12° bis 18°, insbesondere von 13° bis 17,5°, insbesondere von 14° bis 17°, insbesondere 16°, einschließt.

18. Medizinische Vorrichtung nach einem der vorhergehenden Ansprüche,
**dadurch gekennzeichnet, dass**
die Verbinderachse (21) bei einem Expansionsgrad von 80% bis 90%, insbesondere von 82% bis 90%, insbesondere von 84% bis 89,5%, insbesondere von 86% bis 89%, insbesondere 88,9%, mit der Längsachse (15) der Gitterstruktur (10) einen Winkel von 5° bis 12°, insbesondere von 6° bis 11°, insbesondere von 7° bis 10°, insbesondere 8°, einschließt.

19. Medizinische Vorrichtung nach einem der vorhergehenden Ansprüche,
**dadurch gekennzeichnet, dass**
die Gitterstruktur (10) ein superelastisches Material, insbesondere eine Nickel-Titan-Legierung, vorzugsweise Nitinol, aufweist oder daraus besteht.

20. Medizinische Vorrichtung nach einem der vorhergehenden Ansprüche,
**dadurch gekennzeichnet, dass**
die Gitterstruktur (10) im Herstellzustand einen Querschnittsdurchmesser aufweist, der zwischen 3,5 mm und 6 mm, insbesondere 3,5 mm oder 4,5 mm oder 6 mm, beträgt.

21. Medizinische Vorrichtung nach einem der Ansprüche 9 bis 20,
**dadurch gekennzeichnet, dass**
die Gitterstruktur (10) in Umfangsrichtung jeweils zwischen 3 und 6, insbesondere zwischen 3 und 9, insbesondere 6, unmittelbar benachbarte Zellen (30) aufweist, die einen Zellenring (34) bilden und jeweils durch vier Stege (11, 12, 13, 14) begrenzt sind, von denen der erste und dritte Steg (11, 13) jeweils eine erste Stegbreite und der zweite und vierte Steg (12, 14) jeweils eine zweite Stegbreite aufweisen, wobei ein Verhältnis zwischen der ersten Stegbreite und der zweiten Stegbreite zwischen 1:1,1 und 1:1,5, insbesondere zwischen 1:1,1 und 1:1,3, vorzugsweise 1:1,3, beträgt.

22. Medizinische Vorrichtung nach einem der Ansprüche 9 bis 20,
**dadurch gekennzeichnet, dass**
die Gitterstruktur (10) in Umfangsrichtung jeweils mehr als 12, insbesondere zwischen 12 und 48, unmittelbar benachbarte Zellen (30) aufweist, die einen Zellenring (34) bilden und jeweils durch vier Stege (11, 12, 13, 14) begrenzt sind, von denen der erste und dritte Steg (11, 13) jeweils eine erste Stegbreite und der zweite und vierte Steg (12, 14) jeweils eine zweite Stegbreite aufweisen, wobei ein Verhältnis zwischen der ersten Stegbreite und der zweiten Stegbreite zwischen 1:1,1 und 1:3, insbesondere zwischen 1:1,25 und 1:1,5, vorzugsweise 1:1,2, beträgt.

## Claims

1. A medical device for inserting into a hollow organ of the body, comprising a compressible and expandable mesh structure (10) consisting of webs (11, 12, 13, 14), which are interconnected in one piece by web connectors (20), and bordering closed cells (30) of the mesh structure (10), wherein the cells (30) are each bordered by four webs (11, 12, 13, 14) and have a diamond-shaped basic shape in the as-manufactured state, wherein the web connectors form intersections of the webs (11, 12, 13, 14) and each have a connector axis (21), which extends between two cells (30) adjacent to one another in the longitudinal direction of the mesh structure (10), and wherein the mesh structure (10) is self-expandable,
**characterized in that**,
in the transition of the mesh structure (10) from the as-manufactured state into a compressed state, the web connectors (20) rotate in such a manner that an angle between the connector axis (21) and a longitudinal axis (15) of the mesh structure (10) changes, in particular increases, in the transition of the mesh structure (10) from a completely expanded as-manufactured state into a partially expanded intermediate state.

2. The medical device according to claim 1,
**characterized in that**
the angle between the connector axis (21) and the longitudinal axis (15) in the as-manufactured state and/or in a compressed, as-supplied state of the mesh structure (10) amounts to at most 5°, in particular at most 4°, in particular at most 3°, in particular at most 2°, in particular at most 1°.

3. The medical device according to claim 1 or 2,
**characterized in that**
the connector axis (21) in the as-manufactured state and/or in a compressed as-supplied state of the mesh structure (10) is aligned in parallel with the longitudinal axis (15) of the mesh structure (10).

4. The medical device according to any one of the preceding claims,
**characterized in that**
the web connector (20) changes its direction of rotation in the transition of the mesh structure (10) from the as-manufactured state to the compressed state, in particular to the as-supplied state.

5. The medical device according to any one of the preceding claims,
**characterized in that**
the connector axis (21) corresponds to the shortest distance between two cell tips (31, 32) of cells (30) opposite one another in the longitudinal direction on the web connector (20).

6. The medical device according to any one of the preceding claims,
**characterized in that**
cells (30) of the mesh structure (10) adjacent to one another in the longitudinal direction each comprise a cell tip (31, 32) having a curvature (33), wherein the curvature (33) has a maximum, in particular a vertex, which forms an end point (22) of the connector axis (21).

7. The medical device according to any one of the preceding claims,
**characterized in that**
the connector axis (21) intersects the longitudinal axis (15) of the mesh structure (10) at a point of intersection which forms a point of rotation (16) about which the web connector (20) rotates at least in a partially expanded state of the mesh structure (10).

8. The medical device according to claim 7,
**characterized in that**
the web connector (20) is designed with point symmetry based on the point of rotation (16).

9. The medical device according to any one of the preceding claims,
**characterized in that**
four webs (11, 12, 13, 14) are each disposed on a web connector (20), wherein a first web and a third web (11, 13) and a second web and a fourth web (12, 14) are each arranged diametrically opposite the others.

10. The medical device according to claim 9,
**characterized in that**
one neutral fiber (17) of both the first and third webs (11, 13) is perpendicular to the neutral fibers (17) of the second and fourth webs (12, 14).

11. The medical device according to claim 10,
**characterized in that**
the neutral fibers (17) of the second and fourth webs (12, 14) are aligned with one another, and the neutral fibers (17) of the first and third webs (11, 13) are arranged so they are offset from one another.

12. The medical device according to any one of claims 9 to 11,
**characterized in that**
the first and third webs (11, 13) each have a broadened root (18).

13. The medical device according to any one of claims 9 to 12,
**characterized in that**
the first and third webs (11, 13) have an S-shaped course in the area of the web connector (20), and/or the second and fourth webs (12, 14), in particular their neutral fibers (17), have an essentially linear course.

14. The medical device according to any one of the preceding claims,
**characterized in that**
the web connector (20) has a waisted shape.

15. The medical device according to any one of the preceding claims,
**characterized in that**
the connector axis (21) is aligned, preferably in parallel, with the longitudinal axis (15) of the mesh structure (10) at a degree of expansion of the mesh structure (10) of 5% to 15%, in particular of 8% to 12%, in particular of 10% or 9.9%, and/or at a degree of expansion of at least 90%, in particular at least 95%, in particular at least 98%, in particular 100%, each at an angle of at most 5°, in particular at most 4°, in particular at most 3°, in particular at most 2°, in particular at most 1°.

16. The medical device according to any one of the preceding claims,
**characterized in that**
the connector axis (21) forms an angle of 15° to 20°, in particular of 16° to 19°, in particular 18°, with the longitudinal axis (15) of the mesh structure (10) at a degree of expansion of 40% to 50%, in particular of 42% to 48%, in particular 44.4%.

17. The medical device according to any one of the preceding claims,
**characterized in that**
the connector axis (21) forms an angle of 12° to 18°, in particular of 13° to 17.5°, in particular of 14° to 17°, in particular of 16°, with the longitudinal axis (15) of the mesh structure (10), at a degree of expansion of 60% to 70%, in particular of 62% to 68%, in particular 66.7%.

18. The medical device according to any one of the preceding claims,
**characterized in that**
the connector axis (21) forms an angle of 5° to 12°, in particular of 6° to 11°, in particular of 7° to 10°, in particular of 8°, with the longitudinal axis (15) of the mesh structure (10) at a degree of expansion of 80% to 90%, in particular of 82% to 90%, in particular of 84% to 89.5%, in particular of 86% to 89%, in particular of 88.9%.

19. The medical device according to any one of the preceding claims,
**characterized in that**
the mesh structure (10) comprises or is made of a superelastic material, in particular a nickel-titanium alloy, preferably Nitinol.

20. The medical device according to any one of the preceding claims,
**characterized in that**
the mesh structure (10) in the as-manufactured state has a cross-sectional diameter corresponding to between 3.5 mm and 6 mm, in particular 3.5 mm or 4.5 mm or 6 mm.

21. The medical device according to any one of claims 9 to 20,
**characterized in that**
the mesh structure (10) has between 3 and 6, in particular between 3 and 9, in particular 6, directly adjacent cells (30) in the circumferential direction, each forming a cell ring (34) and each bordered by four webs (11, 12, 13, 14), the first and third webs (11, 13) of which each having a first web width and the second and fourth webs (12, 14) each having a second web width, wherein a ratio between the first web width and the second web width is between 1:1.1 and 1:1.5, in particular between 1:1.1 and 1:1.3, preferably 1:1.3.

22. The medical device according to any one of claims 9 to 20,
**characterized in that**
the mesh structure (10) has more than 12, in particular between 12 and 48, directly adjacent cells (30) in the circumferential direction, each forming a cell ring (34) and each being bordered by four webs (11, 12, 13, 14), the first and third webs (11, 13) of which each having a first web width and the second and fourth webs (12, 14) of which each having a second web width, wherein a ratio between the first web width and the second web width is between 1:1.1 and 1:3, in particular between 1:1.25 and 1:1.5, preferably 1:1.2.

## Revendications

1. Dispositif médical destiné à être introduit dans un organe creux du corps, comprenant une structure grillagée comprimable et expansible (10) composée de traverses (11, 12, 13, 14) qui sont reliées entre elles de manière à former un seul bloc par des connecteurs de traverses (20), les cellules (30) étant limitées respectivement par quatre traverses (11, 12, 13, 14) et présentant, en état de fabrication, une forme générale en forme de chenille, les connecteurs de traverses constituant des points de croisement des traverses (11, 12, 13, 14) et présentant respectivement un axe de connexion (21) qui s'étend entre deux cellules (30) voisines dans le sens longitudinal de la structure grillagée (10), et la structure grillagée (10) étant auto-expansible,
**caractérisé en ce que**
les connecteurs de traverses (20), lors du passage de la structure grillagée (10) de l'état de fabrication à un état comprimé, tournent de manière à ce qu'un angle entre l'axe de connecteur (21) et un axe longitudinal (15) de la structure grillagée (10) change, en particulier, augmente, lors du passage de la structure grillagée (10) d'un état de fabrication entièrement expansé à un état intermédiaire partiellement expansé.

2. Dispositif médical selon la revendication 1,
**caractérisé en ce que**
l'angle entre l'axe de connecteur (21) et l'axe longitudinal (15), en état de fabrication et/ou dans un état d'acheminement comprimé de la structure grillagée (10), est au plus de 5°, en particulier au plus de 4°, en particulier au plus de 3°, en particulier au plus de 2°, en particulier au plus de 1°.

3. Dispositif médical selon la revendication 1 ou 2,
**caractérisé en ce que**
l'axe de connecteur (21), en état de fabrication et/ou dans un état d'acheminement comprimé de la structure grillagée (10), est orienté parallèlement à l'axe longitudinal (15) de la structure grillagée (10).

4. Dispositif médical selon une des revendications précédentes,
**caractérisé en ce que**
le connecteur de traverses (20), lors du passage de la structure grillagée (10) de l'état de fabrication à l'état comprimé, en particulier l'état d'acheminement, change de sens de rotation.

5. Dispositif médical selon une des revendications précédentes,
**caractérisé en ce que**
l'axe de connecteur (21) correspond à la distance la plus courte entre deux pointes de cellules (31, 32) de cellules (30) opposées dans le sens longitudinal au niveau du connecteur de traverses (20).

6. Dispositif médical selon une des revendications précédentes,
**caractérisé en ce que**
les cellules (30) voisines dans le sens longitudinal de la structure grillagée (10) comprennent respectivement une pointe de cellule (31, 32) dotée d'une courbure (33), la courbure (33) présentant un maximum, en particulier un point sommital, qui constitue un point terminal (22) de l'axe de connecteur (21).

7. Dispositif médical selon une des revendications précédentes,
**caractérisé en ce que**
l'axe de connecteur (21), du moins dans un état partiellement expansé de la structure grillagée (10), coupe l'axe longitudinal (15) de la structure grillagée (10) à un point de coupe qui constitue un point de rotation (16) autour duquel le connecteur de traverses (20) tourne.

8. Dispositif médical selon la revendication 7,
**caractérisé en ce que**
le connecteur de traverses (20) a une conformation symétrique au point par rapport au point de rotation (16).

9. Dispositif médical selon une des revendications précédentes,
**caractérisé en ce que**
respectivement quatre traverses (11, 12, 13, 14) sont disposées au niveau d'un connecteur de traverses (20), respectivement une première et une troisième traverses (11, 13) et une deuxième et une quatrième traverses (12, 14) étant disposées diamétralement opposées.

10. Dispositif médical selon la revendication 9,
**caractérisé en ce que**
respectivement une fibre neutre (17) des première et troisième traverses (11, 13) est posée verticalement sur les fibres neutres (17) des deuxième et quatrième traverses (12, 14).

11. Dispositif médical selon la revendication 10,
**caractérisé en ce que**
les fibres neutres (17) des deuxième et quatrième traverses (12, 14) sont disposées alignées les unes par rapport aux autres et les fibres neutres (17) des première et troisième traverses (11, 13) sont disposées décalées les unes par rapport aux autres.

12. Dispositif médical selon une des revendications 9 à 11,
**caractérisé en ce que**
les première et troisième traverses (11, 13) présentent respectivement une racine élargie (18).

13. Dispositif médical selon une des revendications 9 à 12,
**caractérisé en ce que**
les première et troisième traverses (11, 13) présentent de c., au niveau du connecteur de traverses (20), une extension en forme de S et/ou que les deuxième et quatrièmes traverses (12, 14), en particulier leurs fibres neutres (17), présentent une extension sensiblement rectiligne.

14. Dispositif médical selon une des revendications précédentes,
**caractérisé en ce que**
le connecteur de traverses (20) présente une forme cintrée.

15. Dispositif médical selon une des revendications précédentes,
**caractérisé en ce que**
l'axe de connecteur (21), à un degré d'expansion de la structure grillagée (10) de 5 % à 15 %, en particulier de 8 % à 12 %, en particulier de 10 % ou 9,9 %, et/ou à un degré d'expansion d'au moins 90 %, en particulier d'au moins 95 %, en particulier d'au moins 98 %, en particulier de 100 %, est orienté respectivement suivant un angle d'au plus 5°, en particulier au plus de 4°, en particulier au plus de 3°, en particulier au plus de 2°, en particulier au plus de 1°, de préférence parallèlement, à l'axe longitudinal (15) de la structure grillagée (10).

16. Dispositif médical selon une des revendications précédentes,
**caractérisé en ce que**
l'axe de connecteur (21), à un degré d'expansion de 40 % à 50 %, en particulier de 42 % à 48 %, en particulier de 44,4 %, décrit avec l'axe longitudinal (15) de la structure grillagée (10) un angle de 15° à 20°, en particulier de 16° à 19°, en particulier de 18°.

17. Dispositif médical selon une des revendications précédentes,
**caractérisé en ce que**
l'axe de connecteur (21), à un degré d'expansion de 60 % à 70 %, en particulier de 62 % à 68 %, en particulier de 66,7 %, décrit avec l'axe longitudinal (15) de la structure grillagée (10) un angle de 12° à 18°, en particulier de 13° à 17,5°, en particulier de 14° à 17°, en particulier de 16°.

18. Dispositif médical selon une des revendications précédentes,
**caractérisé en ce que**
l'axe de connecteur (21), à un degré d'expansion de 80 % à 90 %, en particulier de 82 % à 90 %, en particulier de 84 % à 89,5 %, en particulier de 86 % à 89 %, en particulier de 88,9 %, décrit avec l'axe longitudinal (15) de la structure grillagée (10) un angle de 5° à 12°, en particulier de 6° à 11°, en particulier de 7° à 10°, en particulier de 8°.

19. Dispositif médical selon une des revendications précédentes,
**caractérisé en ce que**
la structure grillagée (10) présente un matériau super-élastique, en particulier un alliage de nickel et titane, de préférence du Nitinol, ou en est composée.

20. Dispositif médical selon une des revendications précédentes,
**caractérisé en ce que**
la structure grillagée (10) présente, en état de fabrication, un diamètre de section transversale qui est compris entre 3,5 mm et 6 mm, ou est en particulier de 3,5 mm ou 4,5 mm ou 6 mm.

21. Dispositif médical selon une des revendications 9 à 20,
**caractérisé en ce que**
la structure grillagée (10) présente dans le sens circonférentiel respectivement entre 3 et 6, en particulier entre 3 et 9, en particulier 6, cellules directement voisines (30) qui forment un anneau de cellules (34) et sont respectivement limitées par quatre traverses (11, 12, 13, 14) dont les première et troisième traverses (11, 13) présentent respectivement une première largeur de traverse et les deuxième quatrième traverses (12, 14) respectivement une seconde largeur de traverse, un rapport entre la première largeur de traverse et la deuxième largeur de traverse étant de 1:1,1 et 1:1,5, en particulier étant compris entre 1:1,1 et 1:1,3, de préférence 1:1,3.

22. Dispositif médical selon une des revendications 9 à 20,
**caractérisé en ce que**
la structure grillagée (10) présente dans le sens circonférentiel respectivement plus de 12, en particulier entre 12 et 48, cellules directement voisines (30) qui forment un anneau de cellules (34) et sont limitées respectivement par quatre traverses (11, 12, 13, 14) dont les première et la troisième traverses (11, 13) présentent respectivement une première largeur de traverse et les deuxième et quatrième traverses (12, 14) respectivement une deuxième largeur de traverse, un rapport entre la première largeur de traverse et la deuxième largeur de traverse étant compris entre 1:1,1 et 1:3, en particulier entre 1:1,25 et 1:1,5, et étant de préférence de 1:1,2.
